# FASCICULE DE BREVET EUROPEEN

(11) **EP 2 566 976 B1**
(45) Date de publication et mention de la délivrance du brevet: **11.11.2015**
(21) Numéro de dépôt: 11717590.1
(22) Date de dépôt: 28.04.2011
(51) Int. Cl.: C12Q 1/04, C12Q 1/68

(54) **TROUSSE DE DIAGNOSTIC ET PROCÉDÉ POUR DÉTECTER UN MICROORGANISME DANS UN ÉCHANTILLON COMPRENANT UN CONTRÔLE EXOGÈNE COLORÉ**
DIAGNOSE-KIT UND VERFAHREN FÜR DEN NACHWEIS EINES MIKROORGANISMUS IN EINER PROBE MIT EINER GEFÄRBTEN EXOGENEN KONTROLLE
DIAGNOSIS KIT AND METHOD FOR DETECTING A MICROORGANISM IN A SAMPLE INCLUDING A COLORED EXOGENOUS CONTROL

(30) Priorité: 04.05.2010 FR 1053462
(43) Date de publication de la demande: 13.03.2013
(73) Titulaire: AES Chemunex, 35270 Combourg (FR)
(72) Inventeur: BERTRAND, Emmanuel, 22520 Binic (FR); BLANCHARD, Béatrice, 22190 Plerin (FR)
(74) Mandataire: Regimbeau
(86) Numéro de dépôt international: PCT/EP2011/056726
(87) Numéro de publication internationale: WO 2011/138219

(56) Documents cités:
- EP-A1- 1 426 447
- US-A1- 2007 015 169
- SAKAMOTO K ET AL: "A RELIABLE METHOD FOR DETECTION AND IDENTIFICATION OF BEER-SPOILAGE BACTERIA WITH INTERNAL POSITIVE CONTROL PCR (IPC-PCR)", JOURNAL OF THE INSTITUTE OF BREWING, INSTITUTE OF BREWING. LONDON, GB, vol. 103, no. 3, 1 janvier 1997 (1997-01-01), page 161, XP008039820, ISSN: 0046-9750
- BEZOLD G ET AL: "Detection of herpes simplex virus and varicella-zoster virus in clinical swabs: frequent inhibition of PCR as determined by internal controls.", MOLECULAR DIAGNOSIS : A JOURNAL DEVOTED TO THE UNDERSTANDING OF HUMAN DISEASE THROUGH THE CLINICAL APPLICATION OF MOLECULAR BIOLOGY DEC 2000 LNKD- PUBMED:11172491, vol. 5, no. 4, décembre 2000 (2000-12), pages 279-284, XP002611063, ISSN: 1084-8592

### SEQUENCE LISTING

<110> AES CHEMUNEX
   BERTRAND, Emmanuel
   BLANCHARD, Béatrice
<120> Trousse de diagnostic et procédé pour détecter un microorganisme dans un échantillon comprenant un contrôle exogène coloré
<130> D28525
<150> FR 1053462
   <151> 2010-05-04
<160> 2
<170> PatentIn version 3.3
<210> 1
   <211> 24
   <212> DNA
   <213> Artificial
<220>
   <223> Forward primer EPC1
<400> 1
   aaaagtgaga ggcacctgtc agat 24
<210> 2
   <211> 20
   <212> DNA
   <213> Artificial
<220>
   <223> Reverse primer EPC2
<400> 2
   atactgctcc tccccgcaat 20

## Description

La présente invention concerne des trousses de diagnostic et des procédés pour la détection d'un microorganisme dans un échantillon incorporant une validation des différentes étapes réactionnelles à l'aide d'un contrôle d'extraction exogène combiné à un indicateur coloré de pH.

Mise au point par K. Mullis en 1985 (Prix Nobel dès 1993), la technique PCR (Polymerase Chain Reaction) connaît un essor considérable dès les années 1990 dans le domaine de la recherche puis du diagnostic. Elle est actuellement utilisée tant dans les domaines de diagnostic clinique, vétérinaire, environnemental qu'agro-alimentaire. Les tests basés sur la PCR ou sur des méthodes d'amplification dérivées de la PCR permettent de détecter une séquence d'acide nucléique dans un échantillon biologique avec une extraordinaire sensibilité et spécificité.

Dans certains domaines, la PCR est aujourd'hui réalisée dans des laboratoires non-spécialistes en biologie moléculaire grâce à l'utilisation de trousses commerciales (ou « kits ») prêtes à l'emploi et simples d'utilisation. Malgré l'existence de telles trousses commerciales facilitant grandement la réalisation de réactions d'amplification complexes, il existe un besoin important de simplification de ces trousses et de validation des manipulations effectuées afin de diminuer le nombre d'erreurs et de rendre l'utilisation de ces trousses plus aisée. De tels contrôles seraient particulièrement utiles lorsque la détection du microorganisme dans l'échantillon implique des étapes d'extraction des acides nucléiques préalables à l'amplification des acides nucléiques spécifiques du microorganisme cible, nécessitant des manipulations et mélanges successifs de différents réactifs. Une telle succession d'étapes réactionnelles peut conduire à des erreurs de manipulation et elle est peu rassurante pour l'opérateur qui ne dispose pas au cours de ces manipulations de contrôles lui permettant de valider chaque étape au fur et à mesure du déroulement du test. Ces problèmes sont rencontrés notamment dans le domaine des tests agro-alimentaires et de diagnostic environnemental qui sont habituellement réalisés dans des laboratoires non spécialisés en biologie moléculaire.

Afin de valider les différentes étapes de la réaction, il était connu d'introduire des contrôles positifs ainsi que des contrôles négatifs réalisés de façon concomitante avec l'amplification de l'acide nucléique spécifique du microorganisme cible. Un acide nucléique contrôle est alors amplifié en même temps que la séquence cible permettant de vérifier le bon déroulement des étapes réactionnelles en absence d'amplification et de détection de la séquence cible. Cependant le simple ajout d'un acide nucléique ne permet pas de vérifier le bon fonctionnement de l'étape d'extraction mais seulement la réaction d'amplification. Inversement, la présence d'un contrôle négatif permet en général de valider l'absence de contamination avec des acides nucléiques cibles.

En outre, lors de la mise en oeuvre d'une analyse de type PCR, l'opérateur est amené à manipuler un grand nombre de fois de petits volumes de liquide incolore. La difficulté étant pour l'opérateur d'être certain qu'il a, à chaque étape réactionnelle, ajouté le liquide incolore dans un autre liquide incolore et ainsi de suite. Une des façons de s'assurer que les ajouts de liquide successifs ont été correctement réalisés est d'utiliser pour chacune des étapes des réactifs colorés. Ainsi, il était également connu d'utiliser des colorants ou des réactifs colorés afin de permettre une validation visuelle de certaines étapes pour l'opérateur. Typiquement, afin de vérifier l'ajout d'une enzyme essentielle au déroulement de la réaction, cette dernière peut être mise à disposition dans un tampon coloré, la manipulation d'un liquide coloré étant plus aisée pour l'opérateur.

La demande de brevet US 2007/0015169 décrit des procédés de purification d'acides nucléiques mettant en oeuvre des tampons colorés. La présence de tampons colorés est cependant uniquement destinée au contrôle du bon déroulement des étapes de mélange de réactif et de lyse. Un contrôle des étapes d'amplification n'est pas décrit. Par ailleurs, ces procédés ne comportent pas de contrôle permettant d'exclure l'inhibition de la réaction d'amplification due à l'échantillon.

Ces trousses PCR ne comportent cependant pas de contrôle efficace pour les étapes se déroulant en amont de la réaction d'amplification incluant notamment l'extraction des acides nucléiques à partir d'un échantillon puis le mélange du produit obtenu avec les réactifs d'amplification. L'utilisation de cellules portant le matériel génétique du contrôle interne permet de contrôler d'une part l'efficacité de l'extraction et d'autre part le bon fonctionnement de l'étape d'amplification. En effet de telles cellules vont subir la même lyse que les germes recherchés dans l'échantillon de façon concomitante. Ce système est d'autant plus juste que les produits d'extraction des cellules contrôles et de l'échantillon sont de mêmes natures (acides nucléiques) et servent de base à la réaction commune de PCR. En outre, l'utilisation de liquides colorés permet à l'opérateur de mieux voir et distinguer les différents liquides manipulés mais il n'y a pas de possibilité de vérifier que les ajouts successifs ont été correctement effectués.

La présente invention propose des procédés et des trousses commerciales combinant un indicateur coloré de pH et un contrôle d'extraction et d'amplification exogène. Le contrôle exogène consiste en un microorganisme contrôle incorporant une séquence ADN contrôle dont l'amplification permet de vérifier à la fois le bon déroulement de la préparation de l'échantillon comprenant l'extraction des acides nucléiques et le bon déroulement de la réaction d'amplification. Ce contrôle d'extraction et d'amplification exogène est combiné à un indicateur coloré de pH de telle façon qu'il est non seulement possible de colorer les liquides réactionnels pour une manipulation plus aisée mais aussi de vérifier le bon déroulement des différentes étapes d'extraction et d'amplification en visualisant le ou les virages de l'indicateur coloré de pH.

Avantageusement, l'indicateur coloré de pH n'interfère ni avec la réaction enzymatique d'amplification, ni avec la détection des produits d'amplification à l'aide de fluorophores par exemple.

Avantageusement, l'indicateur coloré de pH est non toxique et il ne présente donc pas de risques particuliers pour l'opérateur.

Avantageusement, l'indicateur coloré de pH change deux fois de couleur au cours du procédé pour un bon suivi de l'ensemble des étapes d'd'extraction et d'amplification.

Avantageusement, la couleur de l'indicateur coloré de pH n'est affectée ni par des changements brusques de températures ni par des températures élevées supérieures à 90°C.

### Listage des séquences

SEQ ID NO. 1 : Amorce sens EPC1 (24 nucléotides)
SEQ ID NO : 2 : Amorce antisens EPC2 (20 nucléotides)

### Résumé de l'invention

L'invention concerne un procédé pour détecter un microorganisme cible dans un échantillon par l'extraction des acides nucléiques dudit microorganisme cible suivie de l'amplification d'un acide nucléique spécifique dudit microorganisme cible dans lequel le déroulement de l'extraction et de l'amplification sont validés par la réalisation concomitante des étapes suivantes :
a) Mise à disposition d'un contenant comprenant, à l'état sec, un indicateur coloré de pH et un microorganisme contrôle incluant un acide nucléique contrôle,
b) Ajout, à l'état liquide, d'un réactif d'extraction ayant un pH inférieur 7 et obtention d'un mélange réactionnel d'extraction ayant la couleur de la forme acide de l'indicateur coloré de pH,
c) Traitement du mélange réactionnel d'extraction pour libérer les acides nucléiques présents dans ledit mélange réactionnel d'extraction,
d) Mélange d' un aliquote dudit mélange réactionnel d'extraction traité avec un réactif d'amplification ayant un pH supérieur à 7 et obtention d'un mélange réactionnel d'amplification ayant la couleur de la forme basique de l'indicateur pH,
e) Amplification de l'acide nucléique contrôle.

Dans un premier mode de réalisation, l'extraction des acides nucléiques dudit microorganisme cible suivie de l'amplification d'un acide nucléique spécifique dudit microorganisme cible est réalisée dans le ou les mêmes contenants que l'extraction des acides nucléiques du microorganisme contrôle et l'amplification de l'acide nucléique contrôle.

Dans un autre mode de réalisation, l'extraction des acides nucléiques dudit microorganisme cible suivie de l'amplification d'un acide nucléique spécifique dudit microorganisme cible est réalisée dans un ou des contenants distincts du ou des contenants mis en oeuvre pour l'extraction des acides nucléiques du microorganisme contrôle et l'amplification de l'acide nucléique contrôle. Dans ce cas, l'extraction des acides nucléiques dudit microorganisme cible suivie de l'amplification d'un acide nucléique spécifique dudit microorganisme cible est réalisé de préférence dans un ou des contenants comprenant l'indicateur coloré de pH.

Dans un mode de réalisation avantageux de l'invention, le contenant comprend, à l'état sec, ledit indicateur coloré de pH, un microorganisme contrôle incluant un acide nucléique contrôle et un agent de conservation choisi parmi le fructose, le glucose, le mannose, l'amidon, le tréhalose, le galactose et le saccharose.

Préférentiellement, le microorganisme contrôle est un microorganisme Gram négatif.

De préférence, l'indicateur coloré de pH a au moins une zone de virage à un pH compris entre le pH du réactif d'extraction et le pH du réactif d'amplification.

Avantageusement, l'indicateur coloré de pH est choisi parmi le rouge crésol et le bleu de bromothymol.

Dans des modes de réalisation avantageux, l'indicateur coloré de pH a au moins une zone de virage à un pH inférieur au pH du réactif d'extraction et au moins une zone de virage à un pH compris entre le pH du réactif d'extraction et le pH du réactif d'amplification.

De préférence, l'amplification de l'acide nucléique contrôle et, le cas échéant de l'acide nucléique spécifique du microorganisme cible est effectuée par une méthode de PCR quantitative mettant en oeuvre des fluorophores.

L'invention concerne aussi des trousses de diagnostic pour détecter un microorganisme cible dans un échantillon comprenant un premier contenant comprenant, à l'état sec, un indicateur coloré de pH, un microorganisme contrôle incluant un acide nucléique contrôle, et au moins un second contenant comprenant un réactif pour l'amplification d'acides nucléiques comprenant une ADN polymérase.

De préférence, ces trousses comprenant en outre un réactif d'extraction comprenant une protéinase.

Avantageusement, l'indicateur coloré de pH est choisi parmi le rouge de crésol et le bleu de bromophénol.

De préférence, ces trousses comprennent en outre des amorces pour l'amplification de l'acide nucléique contrôle.

De préférence, ces trousses comprennent en outre des amorces pour l'amplification d'un acide nucléique spécifique dudit microorganisme cible.

### Description de l'invention

L'invention se rapporte donc à des procédés et à des trousses de diagnostic pour détecter un microorganisme cible dans un échantillon par amplification d'une séquence d'acide nucléique spécifique dudit microorganisme. Ces procédés et trousses mettent en oeuvre un contrôle positif interne pour les étapes d'extraction et d'amplification ainsi qu'un indicateur coloré de pH dont le virage permet de suivre les ajouts successifs de liquides réactionnels.

L'invention se rapporte plus particulièrement à un procédé pour détecter un microorganisme cible dans un échantillon par l'extraction des acides nucléiques dudit microorganisme cible suivie de l'amplification d'un acide nucléique spécifique dudit microorganisme cible dans lequel le déroulement de l'extraction et de l'amplification sont validés par la réalisation concomitante des étapes suivantes :
a) Mise à disposition d'un contenant comprenant, à l'état sec, un indicateur coloré de pH et un microorganisme contrôle incluant un acide nucléique contrôle,
b) Ajout, à l'état liquide, de l'échantillon et d'un réactif d'extraction ayant un pH inférieur à 7 et obtention d'un mélange réactionnel d'extraction ayant la couleur de la forme acide de l'indicateur coloré de pH,
c) Traitement du mélange réactionnel d'extraction pour libérer les acides nucléiques présents dans ledit mélange réactionnel d'extraction,
d) Mélange d' un aliquote dudit mélange réactionnel d'extraction traité avec un réactif d'amplification ayant un pH supérieur à 7 et obtention d'un mélange réactionnel d'amplification ayant la couleur de la forme basique de l'indicateur pH,
e) Amplification de l'acide nucléique contrôle,
dans lequel l'extraction des acides nucléiques dudit microorganisme cible suivie de l'amplification d'un acide nucléique spécifique dudit microorganisme cible est réalisée dans le ou les mêmes contenants que l'extraction des acides nucléiques du microorganisme contrôle et l'amplification de l'acide nucléique contrôle.

Les étapes réactionnelles nécessaires à la détection du microorganisme cible sont donc réalisées de façon concomitante avec la détection de l'acide nucléique contrôle du microorganisme contrôle. La réalisation concomitante de ces étapes réactionnelles s'effectue donc dans « un seul geste », autrement dit, dans une même manipulation pour l'opérateur du test. La détection du microorganisme cible et du microorganisme contrôle peut s'effectuer dans le même tube (PCR multiplex) ou dans des tubes différents. Lorsque les étapes réactionnelles sont réalisées dans des tubes différents, il est préféré que les tubes utilisés pour la détection du microorganisme cible comprennent l'indicateur coloré de pH pour la validation des ajouts et mélanges de réactifs.

La description concerne également l'extraction des acides nucléiques dudit microorganisme cible suivie de l'amplification d'un acide nucléique spécifique du microorganisme cible réalisées dans un ou des contenants distincts du ou des contenants mis en oeuvre pour l'extraction des acides nucléiques du microorganisme contrôle et l'amplification de l'acide nucléique contrôle. Dans ce cas, l'extraction des acides nucléiques dudit microorganisme cible suivie de l'amplification d'un acide nucléique spécifique dudit microorganisme cible est, de préférence, réalisé dans un ou des contenants comprenant l'indicateur coloré de pH.

Par échantillon, on entend notamment tout échantillon susceptible de contenir le microorganisme cible que l'on cherche à détecter. Il peut notamment s'agir d'un échantillon biologique dérivé d'un aliment ou d'un produit alimentaire. On citera en particulier l'eau, les produits laitiers, les viandes, les ovoproduits, les produits de la mer, les produits végétaux et les échantillons d'environnements. Dans d'autres modes de réalisation, l'échantillon est du sang, du plasma, un tissu biologique, un organe biologique ou un fluide corporel.

Typiquement, dans les procédés et trousses de diagnostic de la présente invention, pour déterminer si le microorganisme cible est présent dans l'échantillon, au moins deux étapes distinctes sont nécessaires : une première étape conduisant à l'extraction ou à la libération des acides nucléiques du microorganisme cible et une deuxième étape d'amplification d'un acide nucléique spécifique dudit microorganisme cible.

Dans certains modes de réalisation, l'échantillon pourra être utilisé directement dans les procédés et les trousses de la présente invention. Cela pourra notamment être le cas pour les échantillons se présentant naturellement à l'état liquide tels que l'eau, le lait ou le plasma par exemple. Alternativement, l'échantillon peut faire l'objet d'un traitement préalable tel que par exemple un broyage ou une mise en culture. Ainsi, l'échantillon peut préalablement être placé dans un milieu de culture et incubé à une température donnée pendant un temps donné. Alternativement, un échantillon solide peut être au préalable broyé et transformé de l'état solide à l'état liquide.

Par « microorganisme cible », on entend tout microorganisme pluricellulaire ou unicellulaire et notamment les virus, les bactéries ou encore les levures. Les trousses et les procédés de la présente invention permettent plus particulièrement de détecter des microorganismes pathogènes dans des échantillons variés dont des échantillons alimentaires. Parmi les microorganismes cibles, on pourra notamment citer *Escherichia coli, Salmonelle spp,* les entérobactéries, *Listeria spp, Campylobacter spp, Klesiella spp, Bacillus cereus, Staphylococcus aureus, Enterococcus faecalis, Clostidium spp, Candida spp, Fusarium, Saccharomyces Aspergillus et les enterovirus.* Les trousses de diagnostic et procédés de la présente invention sont susceptibles de détecter tout microorganisme cible dans un échantillon par amplification d'un acide nucléique spécifique dudit microorganisme cible.

Les trousses et procédés de la présente invention combinent un contrôle d'extraction et d'amplification exogène sous la forme d'un microorganisme contrôle comprenant un acide nucléique contrôle avec un indicateur coloré de pH. Le contrôle exogène et l'indicateur coloré de pH sont avantageusement mis à disposition prêts à l'emploi, à l'état sec, dans un contenant dans lequel sera réalisée la première étape d'extraction des acides nucléiques. L'indicateur coloré de pH et le microorganisme contrôle sont présents à l'état desséché ou déshydraté.

Par « contenant », on entend tout ce qui peut contenir un liquide réactionnel et notamment tout type de récipient ou de tube. Dans un mode de réalisation préféré, le contenant est un tube, une plaque multi-puits ou une barrette comprenant une multitude de puits/tubes.

Le contenant utilisé dans les trousses et procédés de la présente invention se présente donc sous la forme d'un tube ou d'un support comprenant au moins un puits au fond duquel est déposé un culot sec coloré comprenant l'indicateur coloré de pH et le microorganisme contrôle.

Dans des modes de réalisation avantageux de l'invention, ledit contenant comprend, à l'état sec, ledit microorganisme contrôle incluant un acide nucléique contrôle, un indicateur pH et un agent de conservation. Cet agent de conservation permet de maintenir et de préserver l'intégrité du microorganisme à l'état sec lorsque cela s'avère nécessaire. L'agent de conservation est choisi parmi: le fructose, le glucose, le mannose, l'amidon, le tréhalose, le galactose et le saccharose. De préférence, le glucose et le tréhalose peuvent être utilisés à une concentration allant de 0,1 à 5M.

Dans les trousses de diagnostic et les procédés selon l'invention, le microorganisme contrôle peut être n'importe quel microorganisme contenant un acide nucléique contrôle. Dans des modes de réalisation préférés, le microorganisme contrôle est un microorganisme Gram négatif.

Dans les procédés selon l'invention, les acides nucléiques du microorganisme contrôle sont libérés puis l'acide nucléique contrôle est amplifié. Ce microorganisme contrôle permet ainsi de disposer d'un contrôle positif exogène à la fois pour l'étape d'extraction des acides nucléiques et pour l'étape d'amplification. Ce contrôle permet de vérifier le bon déroulement à la fois de l'extraction et de l'amplification. Ce contrôle permet d'exclure une inhibition partielle ou totale de l'amplification qui pourrait être par exemple provoqué par l'échantillon lui-même, par une dégradation des acides nucléiques ou par l'omission d'une étape essentielle du procédé. Le microorganisme contrôle permet ainsi une validation du bon déroulement de l'ensemble des étapes réactionnelles. Dans les trousses de diagnostic et les procédés de la présente invention, l'acide nucléique contrôle peut être n'importe quel acide nucléique contenu dans le microorganisme contrôle pouvant être amplifié. Il peut s'agir d'une séquence naturellement présente dans le microorganisme contrôle ou d'une séquence hétérologue introduite par transformation. Dans des modes de réalisation préférées, le microorganisme contrôle est une souche *d'E.coli* et l'acide nucléique contrôle est une séquence de phage lambda. De préférence, le microorganisme contrôle est inactivé par toute méthode appropriée pour empêcher sa croissance au cours de la réalisation du test.

L'ajout d'un indicateur coloré de pH permet à la fois de mieux visualiser les liquides manipulés et de valider les ajouts et mélanges successifs de liquides réactionnels par le ou les virage(s) de l'indicateur coloré.

La combinaison du microorganisme contrôle et de l'indicateur coloré de pH permet de valider visuellement (par changement de couleur des mélanges réactionnels) et par biologie moléculaire, le bon fonctionnement de l'étape d'extraction des acides nucléiques de l'échantillon traité et de son analyse par PCR pour la recherche d'un micro-organisme cible.

Par « indicateur coloré de pH », on entend un composé chimique ayant la capacité de changer de couleur en fonction du pH. La zone de virage réfère au domaine de valeur de pH dans lequel l'indicateur de pH change de couleur. Dans les procédés et les trousses de diagnostic de la présente invention, l'indicateur coloré de pH, le réactif d'extraction et le réactif d'amplification sont choisis de telle façon que l'indicateur pH possède au moins une zone de virage à un pH compris entre le pH du réactif d'extraction et le pH du réactif d'amplification.

Dans les trousses et les procédés selon l'invention, le pH du réactif d'extraction est de préférence inférieur à 3, 4, 5, 6 ou 7.

Dans les trousses et les procédés de la présente invention, le pH du réactif d'amplification est le pH des tampons habituellement utilisés dans les réactions enzymatiques d'amplification d'acides nucléiques avec des ADN polymérases thermostables tel que par exemple la Taq polymérase. Le pH du réactif d'amplification est par exemple compris entre 7 et 10 et de préférence compris entre 7.5 et 9. De préférence, le pH du réactif d'amplification est supérieur à 7, 7.5, 8, ou 9.

Tout indicateur coloré de pH possédant une zone de virage dans un domaine de pH approprié peut être utilisé dans les trousses et procédés de la présente invention. L'indicateur de pH choisi doit rester stable lors de changements brusques de températures et aux températures élevées habituellement mises en oeuvre dans les réactions d'amplifications d'acides nucléiques. Par ailleurs, l'indicateur coloré sélectionné doit interférer ni avec la réaction enzymatique d'amplification ni avec les fluorophores utilisés pour la détection des produits d'amplification notamment dans les techniques de PCR quantitative.

De préférence, l'indicateur coloré de pH est non toxique et il présente des couleurs franches ou vives facilement identifiables par l'opérateur.

Préférentiellement, indicateur coloré de pH est choisi parmi le bleu de bromophénol, le rouge de congo, le vert de bromocrésol, le rouge de methyle, le bleu de bromothymol, le rouge de phénol, le rouge de crésol, le pourpre de m-crésol, le rouge de phénol, le rouge neutre, le bleu de thymol et le pourpre de bromocrésol.

Plus préférentiellement, l'indicateur coloré de pH est choisi parmi le rouge crésol et le bleu de thymol.

Dans les trousses de diagnostic et les procédés de la présente invention, l'indicateur coloré de pH préféré est le rouge de crésol (ortho-crésolsulfonephtaléine). Les couleurs du rouge de crésol sont le rouge et le jaune. Cet indicateur possède une zone de virage à un pH compris entre pH 7.2 à pH 8.8 (virage de jaune à rouge).

Deux changements de couleur du rouge crésol sont visibles pour l'opérateur au cours des différentes étapes du procédé. Le culot comprenant le microorganisme contrôle et le rouge de crésol a une couleur rouge. La solution vire au jaune lors de l'ajout du tampon d'extraction ou de lyse dont le pH est inférieur à 7.2. Après la lyse, un aliquote du mélange d'extraction est mélangé avec un réactif d'amplification. Le rouge de crésol reprend alors sa couleur rouge et l'opérateur observe une coloration rosé du mélange d'amplification.

La concentration en indicateur coloré de pH dans les différents liquides/mélanges réactionnels mis en oeuvre dans les trousses de diagnostic et les procédés de la présente invention est comprise entre 0,1 mg/ml et 50 mg/ml et de préférence entre 5 mg/ml et 20 mg/ml.

Lorsque le réactif d'extraction (et le cas échéant l'échantillon) est ajouté au culot sec comprenant l'indicateur coloré et le microorganisme contrôle, il en résulte, après agitation, un mélange d'extraction ayant la couleur de la forme acide de l'indicateur. En effet, le réactif d'extraction a typiquement un pH acide. Ce réactif d'extraction comprend les composés nécessaires à l'extraction ou à la libération des acides nucléiques présents dans le mélange d'extraction c'est-à-dire les acides nucléiques du microorganisme contrôle (et le cas échéant les acides nucléiques de l'échantillon comprenant éventuellement le microorganisme cible). Ces acides nucléiques peuvent être de l'ADN ou de l'ARN de préférence, il s'agit d'ADN.

Cette première étape peut déjà s'accompagner d'un changement de couleur de l'indicateur.

Dans des modes de réalisation particuliers de l'invention, le réactif d'extraction est typiquement une solution aqueuse comprenant une protéinase ou un mélange de protéinases ainsi que des détergents tels que par exemple du SDS ou du Triton X100.

Après l'ajout du réactif d'extraction, le mélange d'extraction résultant est traité afin d'extraire ou de libérer les acides nucléiques présents dans ce mélange. Cette étape correspond aussi à une étape de lyse des microorganismes et éventuellement de digestion des protéines qui pourraient inhiber la réaction d'amplification. Ce traitement comprend de préférence une extraction thermique. De préférence, ce traitement comprend ainsi une incubation à une température propice à l'activité des protéinases tel que 65°C pendant au moins 5, 10, 15 ou 20 minutes. Puis les protéinases sont inactivées par un chauffage à 95°C pendant au moins 5 minutes.

Après ce traitement, la totalité ou au moins un aliquote du mélange d'extraction traité est mélangé à un réactif d'amplification. Ce réactif d'amplification comprend tous les constituants nécessaires à l'amplification de l'acide nucléique contrôle (et le cas échéant de l'acide nucléique spécifique du microorganisme cible). Ce réactif d'amplification peut ainsi comprendre une ADN polymérase thermostable de type Taq polymérase ainsi que des nucléotides (dNTPs), un tampon et des amorces appropriées. Pour des méthodes basées sur les techniques de PCR en temps réel, le réactif d'amplification peut en outre comprendre des sondes marquées. Le mélange d'amplification obtenu après mélange ou agitation prend la couleur de la forme basique de l'indicateur pH. Ainsi, lors de l'ajout d'un aliquote du mélange d'extraction traité au réactif d'amplification, l'indicateur coloré de pH change de couleur ce qui permet à l'opérateur de vérifier le bon déroulement de cette étape.

Toute méthode d'amplification d'acides nucléiques et plus particulièrement d'ADN peut être utilisée dans les procédés et les trousses de diagnostic de la présente invention. Ces méthodes d'amplification sont de préférence des méthodes de type PCR ou des méthodes dérivées de la PCR. Plus préférentiellement, l'amplification est réalisée par des méthodes de PCR dans lesquelles la détection des produits d'amplification met en oeuvre des fluorophores.

L'invention se rapporte aussi à des trousses de diagnostic pour détecter un microorganisme cible dans un échantillon comprenant un premier contenant comprenant, à l'état sec, un indicateur coloré de pH, un microorganisme contrôle incluant un acide nucléique contrôle, et au moins un second contenant comprenant un réactif pour l'amplification d'acides nucléiques comprenant un réactif pour l'amplification d'acides nucléiques choisi parmi un tampon d'amplification, des dNTPs et une ADN polymérase.

De préférence, les trousses selon la présente invention comprennent un troisième contenant comprenant un réactif d'extraction comprenant une protéinase. Ce réactif comprend de préférence également un détergent choisi parmi le SDS et le Triton X100.

De façon avantageuse, les trousses de diagnostic selon l'invention comprennent également des amorces permettant d'amplifier l'acide nucléique contrôle ainsi que le cas échéant des amorces permettant d'amplifier un acide nucléique spécifique du microorganisme à tester.

La coloration obtenue ne perturbe pas la réaction PCR tant du point de vue enzymatique (PCR, PCR quantitative) que du point de vue lecture de fluorescence. La réaction de PCR permet d'amplifier la cible et le contrôle exogène soit en un même contenant (réaction PCR multiplex) ou séparément (PCR simplex).

La présence d'amplification du contrôle exogène permet de valider le bon fonctionnement de la réaction de PCR.

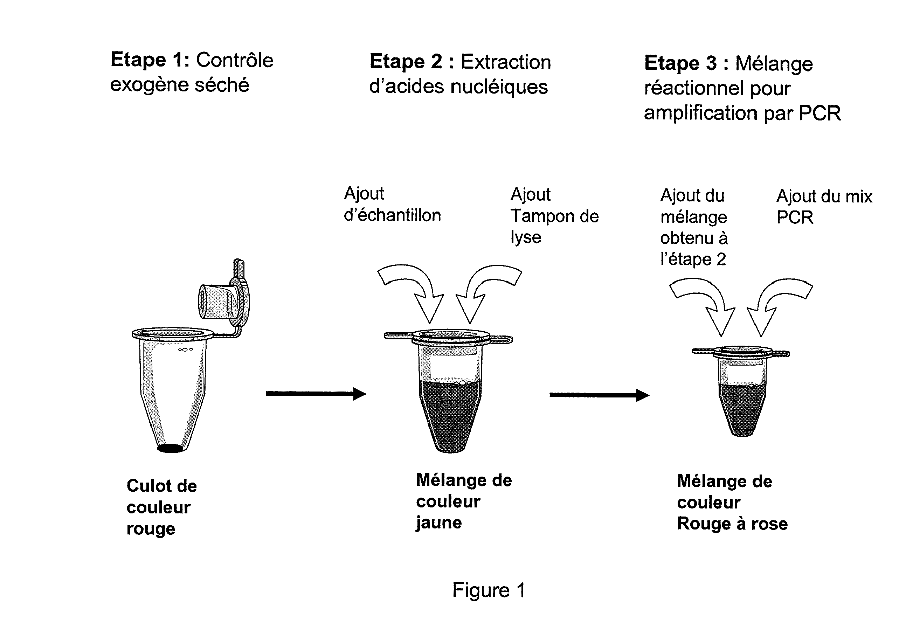

Les procédés selon la présente invention sont illustrés par la figure 1 lorsque l'indicateur est le rouge de crésol. A la première étape, le culot comprenant le contrôle exogène séché avec le carbohydrate et l'indicateur de pH présente une coloration rouge foncé. Il est ajouté sur le culot, de 1 à 500µL d'échantillon (échantillon d'une culture bactérienne) et le tampon de lyse de 5 à 1mL. Le contrôle exogène remis en suspension se trouve alors dans un mélange à pH acide provoquant un changement de couleur de l'indicateur de pH (Couleur jaune). Le lysat obtenu à l'étape 2 est ajouté à un mélange réactionnel de PCR (appelé mix PCR). Le mix PCR présente un pH basique provoquant un changement de couleur (passage du jaune à la couleur rouge) de l'indicateur de pH.

### FIGURES

Figure 1 : Schéma du procédé comprenant l'extraction et l'amplification

### EXEMPLES

### Exemple 1 : préparation du contrôle exogène d'extraction

Le contrôle exogène d'extraction peut être fabriqué de la manière suivante. La nature du contrôle exogène peut être d'origine procaroyte (Escherichia coli, bacillus, ...) ou eucaryote. L'objectif est de transformer un micro-organisme non pathogène avec un fragment d'ADN de séquence chimérique ou identifiée (phage lambda par exemple).

Une séquence de phage lambda (de 100pb à 3000pb) peut être amplifiée par une ADN polymérase polyA (par exemple HotGold Star, Eurogentec) avec un mélange réactionnel suivant : Tampon PCR 1X, MgCl2 2mM, dNTPs 0.2mM, amorces sens EPC1 et antisens EPC2 0.2µM chacune, Taq polymérase 0.2u/réaction, ADN de phage 2µL/réaction. Le programme thermique d'amplification peut être de 10min à 95°C puis de 40 cycles de 30s à 95°C - 30s à 55°C - 30s-3min à 72°C et enfin 7min à 72°C. Le fragment amplifié présente des extensions polyA pour les extrémités 5' et 3'.

Le fragment ainsi obtenu peut être inclus dans un vecteur de type plasmidique (PGEM T) grâce à une enzyme de type ligase (T4 DNA Ligase, Promega). LE mélange suivant peut être réalisé : Tampon de ligation 1X, vecteur 1µL/r, produite de PCR 1µL/r, T4 DNA Ligase 1u/r, Eau qsp 10µL). Une incubation du mélange à 4°C pendant 8h-10h est nécessaire.

Un micro-organisme compétent (E. coli EC219) peut être transformé par le vecteur comprenant la séquence d'intérêt. Cette transformation peut être réalisée par choc thermique par exemple 1min à 42°C puis 5min dans la glace. La sélection des clones est réalisée par un isolement sur une gélose nutritive LB (Lennox Broth par exemple) plus ampicilline (100µg/mL). Les cellules transformées sont cultivées en milieu liquide LB + ampicilline (100µg/mL) à 37°C pendant 16-24h. Une estimation du quorum cellulaire peut être estimée par mesure de densité optique à 600nm et par numération sur milieu gélosé (LB + ampicilline).

Une inactivation des clones est réalisée pour empêcher toute croissance par culture en bouillon LB + ampicilline (100µg/mL) + Ethanol (10% à 35%) à 37°C pendant 3 à 6h.

La suspension de cellules inactivées est diluée en eau physiologique (ou bouillon tryptone sel) de façon à calibrer le nombre de cellules utilisées. La dilution retenue (de 10⁻¹ à 10⁻⁹) est complétée à volume égal avec une solution de carbohydrate (1M à 5M) contenant un indicateur coloré (Rouge de crésol) à 0.1-15mg/mL.

Le mélange est déposé sur support d'extraction (plaque 96 puits, tube individuel, ...) à raison de 1µL à 20µL par puits. Un séchage à 37°C-70°C pendant 30min à 5h est réalisé. Un culot coloré témoigne de la présence du contrôle.

### Exemple 2 : Analyse d'une culture bactérienne par PCR

Une culture bactérienne peut être analysée par PCR suivant la méthode détaillée ci-dessous.

Dix microlitres sont ajoutés à 90µL de tampon de lyse (par exemple : 1-100mg de Pronase ou protéinase K, détergents SDS ou Triton 0.1% à 10%, PVP 1% à 30%) et déposés dans le support d'extraction contenant le contrôle séché. Après agitation, le culot se dissout et provoque une coloration du mélange (Jaune) due aux propriétés de pH acide (1-6.9). L'extraction thermique peut être réalisée par une incubation du mélange à 65°C pendant 20min puis à 95°C pendant 5min.

La coloration n'est pas altérée par les chocs thermiques. Le lysat ainsi obtenu est ajouté à raison de 10 à 20µL à un mix PCR (15-50µL). Le pH basique (7.1-9) du mix PCR provoque un changement de couleur du mélange réactionnel témoignant ainsi de l'ajout d'échantillon.

La coloration du mélange réactionnel PCR ne perturbe pas la réaction PCR tant au niveau de son efficacité que de la lecture de fluorescence.

## Revendications

1. Procédé pour détecter un microorganisme cible dans un échantillon par l'extraction des acides nucléiques dudit microorganisme cible suivie de l'amplification d'un acide nucléique spécifique dudit microorganisme cible dans lequel le déroulement de l'extraction et de l'amplification sont validés par la réalisation concomitante des étapes suivantes :
a. Mise à disposition d'un contenant comprenant, à l'état sec, un indicateur coloré de pH et un microorganisme contrôle incluant un acide nucléique contrôle,
b. Ajout, à l'état liquide, de l'échantillon et d'un réactif d'extraction ayant un pH inférieur 7 et obtention d'un mélange réactionnel d'extraction ayant la couleur de la forme acide de l'indicateur coloré de pH,
c. Traitement du mélange réactionnel d'extraction pour libérer les acides nucléiques présents dans ledit mélange réactionnel d'extraction,
d. Mélange d'un aliquote dudit mélange réactionnel d'extraction traité avec un réactif d'amplification ayant un pH supérieur à 7 et obtention d'un mélange réactionnel d'amplification ayant la couleur de la forme basique de l'indicateur pH,
e. Amplification de l'acide nucléique contrôle,
dans lequel l'extraction des acides nucléiques dudit microorganisme cible suivie de l'amplification d'un acide nucléique spécifique dudit microorganisme cible est réalisée dans le ou les mêmes contenants que l'extraction des acides nucléiques du microorganisme contrôle et l'amplification de l'acide nucléique contrôle.

2. Procédé pour détecter un microorganisme cible dans un échantillon selon la revendication 1 dans lequel ledit contenant comprend, à l'état sec, ledit indicateur coloré de pH, un microorganisme contrôle incluant un acide nucléique contrôle et un agent de conservation choisi parmi le fructose, le glucose, le mannose, l'amidon, le tréhalose, le galactose et le saccharose.

3. Procédé pour détecter un microorganisme cible dans un échantillon selon l'une des revendications 1-2 dans lequel le microorganisme contrôle est un microorganisme Gram négatif.

4. Procédé pour détecter un microorganisme cible dans un échantillon selon l'une des revendications 1-3 dans lequel l'indicateur coloré de pH a au moins une zone de virage à un pH compris entre le pH du réactif d'extraction et le pH du réactif d'amplification.

5. Procédé pour détecter un microorganisme cible dans un échantillon selon l'une des revendications 1-4 dans lequel l'indicateur coloré de pH est choisi parmi le rouge crésol et le bleu de bromothymol.

6. Procédé pour détecter un microorganisme cible dans un échantillon selon l'une des revendications 1-5 dans lequel l'amplification de l'acide nucléique contrôle et, le cas échéant de l'acide nucléique spécifique du microorganisme cible est effectuée par une méthode de PCR quantitative mettant en oeuvre des fluorophores.

7. Trousse de diagnostic pour détecter un microorganisme cible dans un échantillon comprenant un premier contenant comprenant, à l'état sec, un indicateur coloré de pH, un microorganisme contrôle incluant un acide nucléique contrôle, des amorces pour l'amplification d'un acide nucléique spécifique dudit microorganisme cible, et au moins un second contenant comprenant un réactif pour l'amplification d'acides nucléiques comprenant une ADN polymérase.

8. Trousse de diagnostic pour détecter un microorganisme cible dans un échantillon selon la revendication 7 comprenant en outre un réactif d'extraction comprenant une protéinase.

9. Trousse de diagnostic pour détecter un microorganisme cible dans un échantillon selon l'une des revendications 7-8 dans laquelle l'indicateur coloré de pH est choisi parmi le rouge de crésol et le bleu de bromophénol.

10. Trousse de diagnostic pour détecter un microorganisme cible dans un échantillon selon l'une des revendications 7-9 comprenant en outre des amorces pour l'amplification de l'acide nucléique contrôle.

## Patentansprüche

1. Verfahren zum Nachweis eines Ziel-Mikroorganismus in einer Probe durch die Extraktion der Nukleinsäuren des Ziel-Mikroorganismus, gefolgt von der Amplifizierung einer spezifischen Nukleinsäure des Ziel-Mikroorganismus, wobei die Durchführung der Extraktion und der Amplifizierung durch die begleitende Durchführung der folgenden Schritte validiert werden:
a. Bereitstellen eines Behältnisses, das, in trockenem Zustand, einen farbigen pH-Anzeiger und einen Kontroll-Mikroorganismus, der eine Kontroll-Nukleinsäure einschließt, umfasst,
b. Hinzufügen, in flüssigem Zustand, der Probe und eines Extraktionsreagenz mit einem pH unter 7 und Erhalten eines Extraktions-Reaktionsgemischs mit der Farbe der sauren Form des farbigen pH-Indikators,
c. Verarbeiten des Extraktions-Reaktionsgemischs zwecks Freisetzung der in dem Extraktions-Reaktionsgemisch vorhandenen Nukleinsäuren,
d. Mischen eines Aliquots des verarbeiteten Extraktions-Reaktionsgemischs mit einem Amplifizierungsreagenz mit einem pH über 7 und Erhalten eines Amplifizierungs-Reaktionsgemischs mit der Farbe der basischen Form des pH-Indikators,
e. Amplifizieren der Kontroll-Nukleinsäure,
wobei die Extraktion der Nukleinsäuren des Ziel-Mikroorganismus, gefolgt von der Amplifizierung einer spezifischen Nukleinsäure des Ziel-Mikroorganismus, in dem-oder denselben Behältern wie die Extraktion der Nukleinsäuren des Kontroll-Mikroorganismus und die Amplifizierung der Kontroll-Nukleinsäure durchgeführt wird.

2. Verfahren zum Nachweis eines Ziel-Mikroorganismus in einer Probe nach Anspruch 1, wobei der Behälter, in trockenem Zustand, den farbigen pH-Indikator, einen Kontroll-Mikroorganismus, der eine Kontroll-Nukleinsäure einschließt, und ein Konservierungsmittel umfasst, das aus der Fruktose, der Glukose, der Mannose, der Stärke, der Trehalose, der Galaktose und der Saccharose ausgewählt ist.

3. Verfahren zum Nachweis eines Ziel-Mikroorganismus in einer Probe nach einem der Ansprüche 1 bis 2, wobei der Kontroll-Mikroorganismus ein gramnegativer Mikroorganismus ist.

4. Verfahren zum Nachweis eines Ziel-Mikroorganismus in einer Probe nach einem der Ansprüche 1 bis 3, wobei der farbige pH-Indikator mindestens einen Umschlagbereich bei einem pH zwischen inklusive dem pH des Extraktionsreagenz und dem pH des Amplifizierungsreagenz hat.

5. Verfahren zum Nachweis eines Ziel-Mikroorganismus in einer Probe nach einem der Ansprüche 1 bis 4, wobei der farbige pH-Indikator aus dem Kresolrot und dem Bromothymolblau ausgewählt ist.

6. Verfahren zum Nachweise eines Ziel-Mikroorganismus in einer Probe nach einem der Ansprüche 1 bis 5, wobei die Amplifizierung der Kontroll-Nukleinsäure und gegebenenfalls der spezifischen Nukleinsäure des Ziel-Mikroorganismus anhand einer quantitativen PCR-Methode durchgeführt wird, bei der Fluorophore umgesetzt werden.

7. Diagnosekit zum Nachweis eines Ziel-Mikroorganismus in einer Probe, umfassend einen ersten Behälter, der, in trockenem Zustand, einen farbigen pH-Indikator, einen Kontroll-Mikroorganismus, der eine Kontroll-Nukleinsäure einschließt, Primer für die Amplifizierung einer spezifischen Nukleinsäure des Ziel-Mikroorganismus umfasst, und mindestens einen zweiten Behälter, der ein Reagenz für die Amplifizierung von Nukleinsäuren, die eine Polymerase-DNA umfassen, umfasst.

8. Diagnosekit zum Nachweis eines Ziel-Mikroorganismus in einer Probe nach Anspruch 7, das ferner ein Extraktionsreagenz umfasst, das eine Proteinase umfasst.

9. Diagnosekit zum Nachweis eines Ziel-Mikroorganismus in einer Probe nach einem der Ansprüche 7 bis 8, wobei der farbige pH-Indikator aus dem Kresolrot und dem Bromothymolblau ausgewählt ist.

10. Diagnosekit zum Nachweis eines Ziel-Mikroorganismus in einer Probe nach einem der Ansprüche 7 bis 9, das ferner Primer für die Amplifizierung der Kontroll-Nukleinsäure umfasst.

## Claims

1. A method for detecting a target microorganism in a sample by extracting the nucleic acids of said target microorganism and then amplifying a specific nucleic acid of said target microorganism, wherein the advance of the extraction and the advance of the amplification are validated by the concomitant execution of the following steps:
a. providing a container comprising, in the dry state, a colored pH indicator and a control microorganism including a control nucleic acid,
b. adding, in the liquid state, the sample and an extraction reagent having a pH below 7, and obtaining an extraction reaction mixture having the color of the acid form of the colored pH indicator,
c. treating the extraction reaction mixture in order to release the nucleic acids present in said extraction reaction mixture,
d. mixing an aliquot of said extraction reaction mixture treated with an amplification reagent having a pH above 7, and obtaining an amplification reaction mixture having the color of the base form of the pH indicator,
e. amplifying the control nucleic acid,
wherein the extraction of the nucleic acids of said target microorganism and then the amplification of a specific nucleic acid of said target microorganism are carried out in the same container(s) as the extraction of the nucleic acids of the control microorganism and the amplification of the control nucleic acid.

2. The method for detecting a target microorganism in a sample according to claim 1, wherein said container comprises, in the dry state, said colored pH indicator, a control microorganism including a control nucleic acid, and a preservative selected from fructose, glucose, mannose, starch, trehalose, galactose and sucrose.

3. The method for detecting a target microorganism in a sample according to one of claims 1-2, wherein the control microorganism is a Gram-negative microorganism.

4. The method for detecting a target microorganism in a sample according to one of claims 1-3, wherein the colored pH indicator has at least one zone indicating change to a pH between the pH of the extraction reagent and the pH of the amplification reagent.

5. The method for detecting a target microorganism in a sample according to one of claims 1-4, wherein the colored pH indicator is selected from cresol red and bromothymol blue.

6. The method for detecting a target microorganism in a sample according to one of claims 1-5, wherein the amplification of the control nucleic acid and, as the case may be, of the specific nucleic acid of the target microorganism is carried out by a method of quantitative PCR employing fluorophores.

7. A diagnostic kit for detecting a target microorganism in a sample, comprising a first container comprising, in the dry state, a colored pH indicator, a control microorganism including a control nucleic acid, primers for amplifying a specific nucleic acid of said target microorganism, and at least a second container comprising a reagent for amplifying nucleic acids comprising a DNA polymerase.

8. The diagnostic kit for detecting a target microorganism in a sample according to claim 7, further comprising an extraction reagent comprising a proteinase.

9. The diagnostic kit for detecting a target microorganism in a sample according to one of claims 7-8, wherein the colored pH indicator is selected from cresol red and bromophenol blue.

10. The diagnostic kit for detecting a target microorganism in a sample according to one of claims 7-9, further comprising primers for amplifying the control nucleic acid.
